Europäisches Patentamt

European Patent Office

Office européen des brevets

(11) Veröffentlichungsnummer : **0 385 422 B1**

(12) **EUROPÄISCHE PATENTSCHRIFT**

(45) Veröffentlichungstag der Patentschrift :
29.07.92 Patentblatt 92/31

(51) Int. Cl.⁵ : **C07D 471/04, A61K 31/435**

(21) Anmeldenummer : **90103869.5**

(22) Anmeldetag : **28.02.90**

(54) **1,4-Dihydro-5-Isopropoxy-2-Methyl-4-(2-Trifluormethyl-Phenyl)-1,6-Naphthyridin-3-Carbonsäure-(2-(N-Methyl-N-Phenylmethylamino)**

(30) Priorität : **01.03.89 DE 3906406**

(43) Veröffentlichungstag der Anmeldung :
**05.09.90 Patentblatt 90/36**

(45) Bekanntmachung des Hinweises auf die
Patenterteilung :
**29.07.92 Patentblatt 92/31**

(84) Benannte Vertragsstaaten :
**AT BE CH DE DK ES FR GB GR IT LI LU NL SE**

(56) Entgegenhaltungen :
**DE-A- 3 502 790**
**DE-A- 3 602 655**
**DE-A- 3 605 743**

(73) Patentinhaber : **GÖDECKE
AKTIENGESELLSCHAFT
Salzufer 16
W-1000 Berlin 10 (DE)**

(72) Erfinder : **Herrmann, Wolfgang, Dr.
Zum Baumgarten 13
W-7802 Merzhausen (DE)**
Erfinder : **Kleinschroth, Jürgen, Dr.
Ricarda-Huch-Str. 11
W-7819 Denzlingen (DE)**
Erfinder : **Steiner, Klaus, Dr.
Tannenweg 26
W-7808 Waldkirch (DE)**

EP 0 385 422 B1

## Beschreibung

Gegenstand der Erfindung ist (±)-1,4-Dihydro-5-isopropoxy-2-methyl-4-(2-trifluormethylphenyl)-1,6-naphthyridin-3-carbonsäure-[2-(N-methyl-N-phenylmethylamino)ethyl]ester Fumarat sowie die optischen Isomeren (+)-1,4-Dihydro-5-isopropoxy-2-methyl-4-(2-trifluormethylphenyl)-1,6-naphthyridin-3-carbonsäure-[2-(N-methyl-N-phenylmethylamino) ethyl]ester Fumarat und (-)-1,4-Dihydro-5-isopropoxy-2-methyl-4-(2-trifluormethylphenyl)-1,6-naphthyridin-3-carbonsäure-[2-(N-methyl-N-phenylmethylamino)ethyl]ester Fumarat.

In der DE-A-34 31 303 sind 1,6-Naphthyridin-Derivate mit Calcium-antagonistischen Eigenschaften beschrieben, darunter auch (±)-1,4-Dihydro-5-isopropoxy-2-methyl-4-(2-trifluormethylphenyl)-1,6-naphthyridin-3-carbonsäure-[2-(N-methyl-N-phenylmethylamino)-ethyl]ester in der Form des Hydrochlorids.

Überraschenderweise wurde nun gefunden, daß diese Verbindung gegenüber den anderen Derivaten besonders ausgeprägte blutdrucksenkende Wirkungen besitzt. Noch ausgeprägter sind die pharmakologischen Ergebnisse bei den nach üblichen Methoden durch Racemattrennung erhaltenen optischen Isomeren, insbesondere beim (+)-1,4-Dihydro-5-isopropoxy-2-methyl-4-(2-trifluormethylphenyl)-1,6-naphthyridin-3-carbonsäure-[2-(N-methyl-N-phenylmethylamino)-ethyl]ester.

Der Ester liegt jedoch als ölige Base vor und das Hydrochlorid-Salz weist eine Instabilität gegenüber Temperatur- und Lichteinflüssen auf, welches für die galenische Entwicklung und für einen klinischen Einsatz unerwünschte Eigenschaften sind. Schon bei Raumtemperatur ist das Hydrochlorid instabil und ein Streßtest ergab nach 2 Wochen Erhitzen auf 45°C im Dunkeln 10% Zersetzungsprodukte, bei Raumtemperatur am Licht war nach 2 Wochen 30% der Substanz zersetzt. Zudem wurde ein unerwünschter, sehr rascher Wirkungseintritt im Tierversuch gefunden, der auf die gute Löslichkeit der Verbindung in Wasser zurückgeführt wurde.

Aufgabe der Erfindung ist es daher, (+)-1,4-Dihydro-5-isopropoxy-2-methyl-4-(2-trifluormethylphenyl)-1,6-naphthyridin-3-carbonsäure-[2-(N-methyl-N-phenylmethylamino)ethyl]ester in kristalliner, licht- und temperaturstabiler Form für den klinischen Einsatz bereitzustellen, welche zudem noch einen langsameren Wirkungseintritt aufweist.

Überraschenderweise wurde nun gefunden, das das Fumarat als kristallines Salz mit einem Schmelzpunkt von 180 - 182 °C erhalten werden kann. Es ist zudem auch unerwarteterweise unter den oben angeführten Streßbedingungen (2 Wochen 45 °C bzw. 2 Wochen Licht) stabil. Weiterhin weist das Fumarat auch eine erheblich geringere Wasserlöslichkeit auf, wie aus Tabelle 1 ersichtlich ist:

### Tabelle 1

| Löslichkeit in: | Wasser | künstl. Magensaft |
|---|---|---|
| Hydrochlorid | 20% | 20% |
| Fumarat | 0,1% | 1,5% |

Pharmakologiische Versuche an der Ratte ergaben, daß der Wirkungseintritt nach oraler Gabe beim Fumarat etwas später erfolgt als beim Hydrochlorid und zudem überraschenderweise auch die blutdrucksenkende Wirkung beim Fumarat noch wesentlich stärker ist, wie in Tabelle 2 dargestellt:

### Tabelle 2

| Spontan hypertensive Ratte | 1 mg/kg p.o.; n = 6 |
|---|---|
| Hydrochlorid | 17% ± 4,4 |
| Fumarat | 26% ± 2,7 |

Ein weiterer Gegenstand der Erfindung ist die Verwendung der erfindungsgemäßen Verbindungen zur Behandlung von Bluthochdruck, Herz- und Gefäßerkrankungen sowie diese Verbindungen enthaltende Arzneimittel, gegebenenfalls mit den üblichen Hilfs- und Zusatzstoffen. Die erfindungsgemäßen Verbindungen können in flüssiger oder fester Form oral oder parenteral appliziert werden. Als Injektionslösung kommt vor allem Wasser zur Anwendung, welches die bei Injektionslösungen üblichen Zusätze wie Stabilisierungsmittel, Lösungsvermittler oder Puffer enthält.

Derartige Zusätze sind z. B. Tartrat- und Citrat-Puffer, Ethanol, Komplexbildner (wie Äthylendiamin-tetraessigsäure und deren nicht-toxische Salze) sowie hochmolekulare Polymere (wie flüssiges Polyäthylenoxid) zur Viskositätsregulierung. Feste Trägerstoffe sind z. B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Polyäthylenglykol); für orale Applikation geeignete Zübereitungen können gewünschtenfalls zusätzliche Geschmacksund/oder Süßstoffe enthalten.

1,4-Dihydro-5-isopropoxy-2-methyl-4-(2-trifluormethylphenyl)-1,6-naphthyridin-3-carbonsäure -[2-(N-methyl-N-phenylmethylamino)-ethyl]ester Fumarat kann hergestellt werden z.B. durch Zusammengeben der Lösungen von Base und Fumarsäure in geeigneten Lösungsmitteln oder durch Erhitzen der Mischung äquimolarer Mengen von Base und Fumarsäure in einem geeigneten Lösungsmittel und anschließende Kristallisation durch Abkühlen. Als Lösungsmittel sind beispielsweise geeignet Ethylacetat, niedere aliphatische Alkohole und niedere aliphatische Ketone, eventuell in Mischung mit Wasser.

## Beispiel 1

40 g (+) 1,4-Dihydro-5-isopropoxy-2-methyl-4-(2-trifluormethylphenyl)-1,6-naphthyridin-3-carbonsäure-[2-(N-methyl-N-phenylmethylamino)-ethyl]ester werden zusammen mit 8,6 g Fumarsäure unter Rückflußkochen in 1000 ml Essigsäureethylester gelöst. Die Lösung wird filtriert. Das Filtrat wird auf ein Volumen von ca. 180 ml eingeengt. Hierbei fällt ein kristallines Produkt aus. Dieses wird abgesaugt und mit 180 ml Essigsäureethylester gewaschen. Nach dem Trocknen wird das Produkt in 300 ml Ethanol unter leichtem Erwärmen gelöst und danach unter Rühren mit 1500 ml Wasser versetzt. Die Lösung läßt man unter Rühren auf Raumtemperatur abkühlen. Das auskristallisierte Produkt wird abgesaugt und mit 100 ml Wasser nachgewaschen. Das Produkt wird im Vakuum bis zur Gewichtskonstanz getrocknet. Ausbeute: 43,1 g (88,7%); (Fp: 180 - 182 °C; $[\alpha]_D$ = + 3,8 °C (c = 1/MeOH); enantiomere Reinheit 99% (PMR)).

## Beispiel 2

2,9 g (-)1,4-Dihydro-5-isopropoxy-2-methyl-4-(2-trifluormethylphenyl)-1,6-naphthyridin-3-carbonsäure-[2-(N-methyl-N-phenylmethylamino)-ethyl]ester werden zusammen mit 0,58 g Fumarsäure unter Rückflußkochen in 75 ml Essigsäureethylester gelöst. Die Lösung wird filtriert. Das Filtrat wird auf ein Volumen von ca. 20 ml eingeengt. Das ausgefallene, kristalline Produkt wird abgesaugt und mit 10 ml Essigsäureethylester nachgewaschen. Nach dem Trocknen wird das Produkt in 19,5 ml Ethanol unter leichtem Erwärmen gelöst und danach unter Rühren mit 110 ml Wasser versetzt. Die Lösung läßt man unter Rühren auf Raumtemperatur abkühlen. Das auskristallisierte Produkt wird abgesaugt und mit 20 ml Wasser nachgewaschen. Das Produkt wird im Vakuum bis zur Gewichtskonstanz getrocknet. Ausbeute: 2,1 g (64,2%); (Fp: 181-183°C; $[\alpha]_D$ = - 3,6° (c = 1/MeOH); enantiomere Reinheit 99% (PMR).

## Patentansprüche

1. (±)-1,4-Dihydro-5-isopropoxy-2-mehthyl-4-(2-trifluormethylphenyl)-1,6-naphthyridin-3-carbonsäure-[2-(N-methyl-N-phenylmethylamino)ethyl]ester Fumarat sowie seine optischen Isomeren (+)-1,4-Dihydro-5-isopropoxy-2-methyl-4-(2-trifluormethylphenyl)-1,6-naphthyridin-3-carbonsäure-[2-(N-methyl-N-phenylmethyla mino)ethyl]ester Fumarat und (-)-1,4-Dihydro-5-isopropoxy-2-methyl-4-(2-trifluormethylphenyl)-1,6-naphthyridin-3-carbonsäure-[2-(N-methyl-N-phenylmethylamino) ethyl]ester Fumarat.

2. Verfahren zur Herstellung von (±)-1,4-Dihydro-5-isopropoxy-2-methyl-4-(2-trifluormethylphenyl)-1,6-naphthyridin-3-carbonsäure-[2-(N-methyl-N-phenylmethylamino)ethyl]ester Fumarat sowie seinen optischen Isomeren (+)-1,4-Dihydro-5-isopropoxy-2-methyl-4-(2-trifluormethylphenyl)-1,6-naphthyridin-3-carbonsäure-[2-(N-methyl-N-phenylmethylamino)ethyl]ester Fumarat und (-)-1,4-Dihydro-5-isopropoxy-2-methyl-4-(2-trifluormethylp henyl)-1,6-naphthyridin-3-carbonsäure-[-(N-methyl-N-phenylmethylamino)ethyl]ester Fumarat, dadurch gekennzeichnet, daß man entweder Lösungen von Basen der Verbindungen und Fumarsäure in geeigneten Lösungsmitteln zusammengibt oder Base und Fumarsäure in äquimolaren Mengen in geeignetem Lösungsmittel erhitzt und anschließend durch Abkühlen auskristallisiert,

3. Arzneimittel enthaltend Verbindungen gemäß Anspruch 1 und übliche Hilfs- und Zusatzstoffe.

4. Verwendung von Verbindungen gemäß Anspruch 1 zur Herstellung von Arzneimitteln zur Behandlung von Bluthochdruck und Herz- und Gefäßerkrankungen.

5. Verwendung von Verbindungen, hergestellt gemäß Anspruch 2 zur Herstellung von Arzneimitteln zur Behandlung von Bluthochdruck und Herz- und Gefäßerkrankungen.

6. Herstellung von Arzneimitteln, dadurch gekennzeichnet, daß man Arzneimittelwirkstoffe nach Anspruch 1 oder 2 mit üblichen Hilfs- und Zusatzstoffen versetzt.

## Claims

1. (±)-1,4-Dihydro-5-isopropoxy-2-methyl-4-(2-trifluoromethylphenyl)-1,6-naphthyridine-3-carboxylic acid (2-(N-methyl-N-phenylmethylamino)-ethyl] ester fumarate, as well as the optical isomers thereof (+)-1,4-dihydro-5-isopropoxy-2-methyl-4-(2-trifluoromethylphenyl)-1,6-naphthyridine-3-carboxylic acid (2-(N-methyl-N-phenylmethylamino)-ethyl] ester fumarate and (-)-1,4-dihydro-5-isopropoxy-2-methyl-4-(2-trifluoromethylphenyl)-1,6-naphthyridine-3-carboxylic acid (2-(N-methyl-N-phenylmethylamino)-ethyl] ester fumarate.

2. Process for the preparation of (±)-1,4-dihydro-5-isopropoxy-2-methyl-4-(2-trifluoromethylphenyl)-1,6-naphthyridine-3-carboxylic acid (2-(N-methyl-N-phenylmethylamino)-ethyl] ester fumarate, as well as the optical isomers thereof (+)-1,4-dihydro-5-isopropoxy-2-methyl-4-(2-trifluoromethyl-phenyl)-1,6-naphthyridine-3-carboxylic acid [2-(N-methyl-N-phenylmethylamino)-ethyl] ester fumarate and (-)-1,4-dihydro-5-isopropoxy-2-methyl-4-(2-trifluoromethylphenyl)-1,6-naphthyridine-3-carboxylic acid [-(N-methyl-N-phenylmethylamino)-ethyl] ester fumarate, characterised in that either solutions of bases of the compounds and fumaric acid in appropriate solvents are mixed together or the base and fumaric acid in equimolar amounts are heated in an appropriate solvent and there is subsequent crystallisation by cooling.

3. Pharmaceutical compositions containing compounds according to claim 1 and conventional adjuvant and additive materials.

4. The use of compounds according to claim 1 for the preparation of pharmaceutical compositions for the treatment of high blood pressure and of heart and blood vessel diseases.

5. The use of compounds prepared according to claim 2 for the preparation of pharmaceutical compositions for the treatment of high blood pressure and of heart and blood vessel diseases.

6. Preparation of pharmaceutical compositions, characterised in that pharmaceutical composition active ingredients according to claim 1 or 2 are mixed with conventional adjuvant and additive materials.

## Revendications

1. Fumarate de l'ester (N-méthyl-N-phénylméthylamino)-2-éthylique de l'acide (±)-dihydro-1,4-isopropoxy-5-méthyl-2-(trifluorométhyl-2-phényl)-4-naphtyridine-1,6-carboxylique ainsi que ses isomères optiques fumarate de l'ester (N-méthyl-N-phénylméthylamino)-2-éthylique de l'acide (+)-dihydro-1,4-isopropoxy-5-méthyl-2-(trifluorométhyl-2-phényl)-4-naphtyridine-1,6-carboxylique et fumarate de l'ester (N-méthyl-N-phénylméthylamino)-2-éthylique de l'acide (-)-dihydro-1,4-isopropoxy-5-méthyl-2-(trifluorométhyl-2-phényl)-4-naphtyridine-1,6-carboxylique.

2. Procédé de préparation de fumarate de l'ester (N-méthyl-N-phénylméthylamino)-2-éthylique de l'acide (±)-dihydro-1,4-isopropoxy-5-méthyl-2-(trifluorométhyl-2-phényl)-4-naphtyridine-1,6-carboxylique ainsi que ses isomères optiques fumarate de l'ester (N-méthyl-N-phénylméthylamino)-2-éthylique de l'acide (+)-dihydro-1,4-isopropoxy-5-méthyl-2-(trifluorométhyl-2-phényl)-4-naphtyridine-1,6-carboxylique et fumarate de l'ester (N-méthyl-N-phénylméthylamino)-2-éthylique de l'acide (-)-dihydro-1,4-isopropoxy-5-méthyl-2-(trifluorométhyl-2-phényl)-4-naphtyridine-1,6-carboxylique, caractérisé en ce que, soit l'on mélange des solutions de bases des dérivés et de l'acide fumarique dans des solvants appropriés, soit l'on chauffe la base et l'acide fumarique en quantité équimolaire dans un solvant approprié et ensuite on cristallise par refroidissement.

3. Médicament contenant des dérivés selon la revendication 1 et adjuvants et usuels.

4. Utilisation de dérivés selon la revendication 1, pour la préparation de médicaments de traitement de l'hypertension et des maladies cardiaques et vasculaires.

5. Utilisation de dérivés préparés selon la revendication 2, pour la préparation de médicaments destinés au traitement de l'hypertension et des maladies cardiaques et vasculaires.

6. Préparation de médicaments caractérisée en ce que des adjuvants et additifs usuels sont ajoutés aux substances médicamenteuses efficaces selon la revendication 1 ou 2.